(19) Europäisches Patentamt
European Patent Office
Office européen des brevets

(11) Publication number: **0 234 931 B1**

(12) # EUROPEAN PATENT SPECIFICATION

(45) Date of publication of the patent specification: **27.06.90**

(21) Application number: **87301637.2**

(22) Date of filing: **25.02.87**

(51) Int. Cl.[5]: **C07D 471/04,** C07D 475/12, C07D 487/04 // A61K31/505 ,(C07D471/04, 241:00, 221:00)

(54) **Azaanthracene compounds.**

(30) Priority: **26.02.86 JP 40915/86**

(43) Date of publication of application: **02.09.87 Bulletin 87/36**

(45) Publication of the grant of the patent: **27.06.90 Bulletin 90/26**

(84) Designated Contracting States: **AT BE CH DE ES FR GB GR IT LI LU NL SE**

(56) References cited:
**EP-A- 0 090 681**
**US-A- 4 296 114**
**US-A- 4 393 064**

**Carl R. Noller "Lehrbuch der Organischen Chemie" 1960 Springer-Verlag, Berlin-Göttingen-Heidelberg pages 255,256**

(73) Proprietor: **TAISHO PHARMACEUTICAL CO. LTD, 24-1 Takata 3-chome Toshima-ku, Tokyo 171(JP)**

(72) Inventor: **Nakagami, Jozi, 37-15, Sakae-2-chome, Inamachi Kitaadachi-gun Saitama-ken(JP)**
Inventor: **Eguchi, Tadashi, 1515-8, Kamitsuchidana, Ayase-shi(JP)**
Inventor: **Amano, Takehiro, 8-8 Ohara-2-chome, Urawa-shi(JP)**
Inventor: **Sota, Kaoru, 1158-11 Shimotomi, Tokorozawa-shi(JP)**
Inventor: **Sakakibara, Jinsaku, 420 Motoyagoto-3-chome, Tenpaku-ku nagoya(JP)**

(74) Representative: **Harrison, David Christopher, MEWBURN ELLIS & CO 2/3 Cursitor Street, London EC4A 1BQ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 234 931 B1

ACTORUM AG

**Description**

BACKGROUND OF THE INVENTION

1. Field of the Invention

The present invention relates to azaanthracene compounds, and more particularly to novel tri- or tetraazaanthracene compounds and the salts thereof having antitumor activity.

2. Description of the Prior Art

Any tri- or tetraazaanthracene compounds having antitumor activity are not known in the past.

Prior art anticancer agents can not be said to be sufficient for their pharmaceutical activity and toxicity.

The structures of the anticancer agents used clinically have actively been modified in order to increase antitumor effect and decrease side-effect. But it is desired to find the anticancers having new skeletones for treatment of the cancer which is not recognized to be effective by the prior art drugs and for solving the problem of drug tolerance.

SUMMARY OF THE INVENTION

As a result of the earnest researches in view of the above circumstances, the inventors have found novel azaanthrancene compounds having high antitumor activity, and the present invention have been completed.

The present invention relates to azaanthracene compounds of the formula (I)

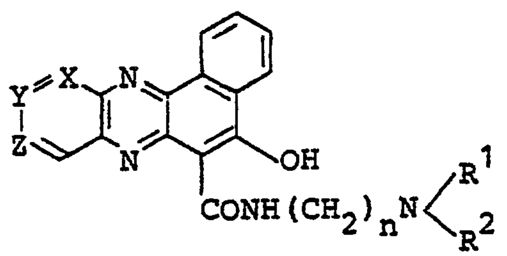

(I)

(wherein one or two of X, Y and Z are nitrogen atoms, the others are CH, $R^1$ and $R^2$ are the same or different and are hydrogen atoms, lower alkyl groups or β-hydroxyethyl groups, and n is 2 or 3) and the salts thereof.

The lower alkyl group in the present invention means an alkyl group such as a methyl group, an ethyl group, a propyl group and a butyl group.

Preferred compounds of formula (I) are those in which one or two of X, Y and Z are nitrogen atoms, the others are CH, $R^1$ and $R^2$ are both methyl groups, or $R^1$ is a hydrogen atom and $R^2$ is a β-hydroxyethyl group, and n is 2, and the salts thereof.

DETAILED DESCRIPTION OF THE INVENTION

Azaanthracene compounds of formula (I) are novel and can be prepared from known ethyl 3-hydroxy-1,4-dihydro-1,4-dioxo-2-naphthoate according to the following reaction scheme (wherein X, Y, Z, $R^1$, $R^2$ and n are as defined above).

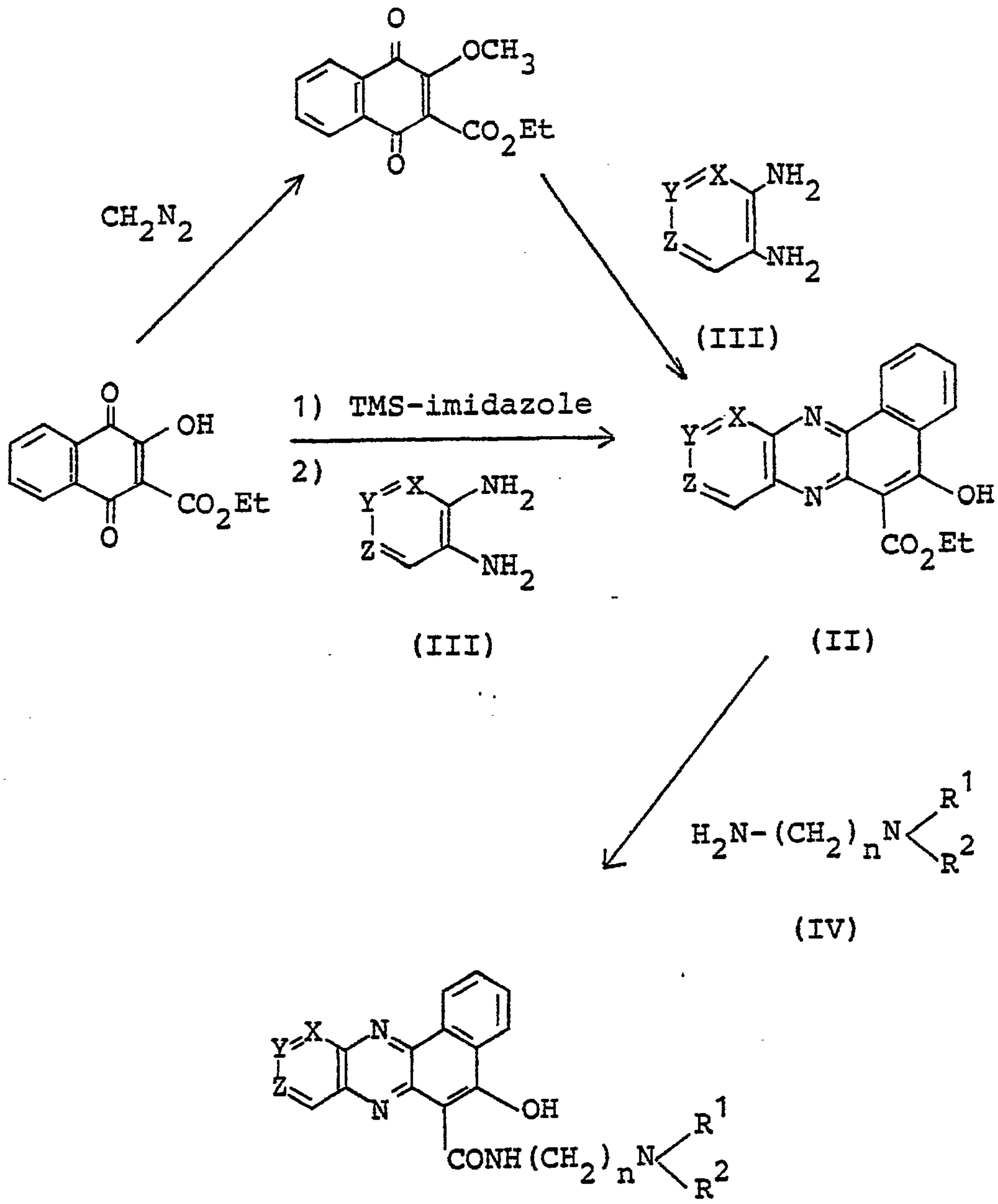


(III)

(III)

(II)

(IV)

(I)

First ethyl 3-hydroxy-1,4-dihydro-1,4-dioxo-2-naphthoate is methylated in an alcohol (e.g., ethanol) or diethyl ether with an excess amount of diazomethane in diethyl ether to give ethyl 3-methoxy-1,4-dihydro-1,4-dioxo-2-naphthoate, which is then condensed with 1.1 to 1.5 molar equivalents of the compound of formula (III) in an organic solvent at room temperature for 2 to 24 hours to give the compound of formula (II). The methylation is carried out at 0°C to room temperature for 2 to 24 hours. Typical examples of the compound of formula (III) are 3,4-diaminopyridine, 2,3-diaminopyridine, 4,5-diaminopyrimidine and the like. Examples of the organic solvent are methanol, ethanol, dimethylformamide, dimethyl sulfoxide, tetrahydrofuran and the like.

Alternatively, ethyl 3-hydroxy-1,4-dihydro-1,4-dioxo-2-naphthoate is reacted with N-trimethylsilylimidazole (TMS-imidazole) in an organic solvent at O°C to room temperature for 2 to 3 hours, and then the resulting compound is condensed with the compound of formula (III) to give the compound of formula (II). Examples of the organic solvent are dimethylformamide, tetrahydrofuran, dichloromethane, chloroform and the like. The condensation is carried out under the same conditions as those of the above condensation.

Next, the compound of formula (II) is reacted with 1.1 to 1.5 molar equivalents of the compound of formula (IV) in an organic solvent at room temperature to 120°C for l to 24 hours to give the compound of formula (I). Typical examples of the compound of formula (IV) are N,N-dimethylethylenediamine, N,N-dimethyl-1,3-propanediamine, 2-(2-aminoethylamino)ethanol and the like. Examples of the organic solvent are benzene, toluene, chloroform, dichloromethane, tetrahydrofuran, dimethylformamide, methanol, ethanol and the like.

The salts of the compound of formula (I) include salts of inorganic acids such as hydrochloric acid, sulfuric acid and the like, and organic acids such as acetic acid, propionic acid, butyric acid, oxalic acid, malic acid, malonic acid, fumalic acid, benzoic acid, toluic acid and the like which are prepared by treating the compounds of formula (I) with the corresponding acids.

The compounds of formula (I) showed antitumor activity on transplanted mouse tumors and are useful for regression or palliation of leukemia in mammals. For the purposes, they may be administered orally, parenterally or locally in conventional dosage forms such as tablets, powders, capsules, solutions, suspensions, emulsions, suppositories and the like, all of which are prepared according to the conventional pharmaceutical practices.

The effective dosage of the compounds of formula (I) depends on the age, weight or response of patient. Generally, however, the daily dosage may range from 0.1 to 10 mg/kg.

Subsequently, the present invention is illustrated in more detail by examples and a test example.

Example 1

(1) Preparation of ethyl 5-hydroxybenzo[f]pyrido [3,4-b]quinoxaline-6-carboxylate:

In 80 ml of ethanol, were dissolved 1.0 g of 3,4-diaminopyridine and 2.3 g of ethyl 1,4-dihydro-1,4-dioxo-3-methoxy-2-naphthoate, and the solution was refluxed for 4 hours. After cooling, the crystals which formed were collected by filtration and recrystallized from ethanol to give the title compound. Yellow needles, m.p. 156-158°C
Yield, 1.4 g

(2) Preparation of N-β-dimethylaminoethyl 5-hydroxybenzo[f]pyrido[3,4-b]quinoxaline-6-carboxamide:

In 50 ml of benzene was dissolved 500 mg of ethyl 5-hydroxybenzo[f]pyrido[3,4-b)quinoxaline-6-carboxylate obtained by synthesis (l) above. 0.l ml of N,N-dimethylethylenediamine was added, and the mixture was refluxed for 8 hours. After cooling, the crystals which formed were collected by filtration and recrystallized from benzene-petroleum ether to give the title compound. Yellow needles, m.p. 178-182°C
Yield, 184 mg
Elementary analysis for $C_{20}H_{19}N_5O_2$
Calcd. (%) C; 66.47, H; 5.30, N; 19.38
Found (%) C; 66.73, H; 5.48, N; 19.37

Example 2

Preparation of N-β-dimethylaminoethyl 5-hydroxybenzo[f]pyrido[3,4-b]quinoxaline-6-carboxamide hydrochloride.

In 200 ml of a mixture of dichloromethane and diethyl ether (1:1) was dissolved 2.06 g of N-β-dimethylaminoethyl 5-hydroxybenzo[f]pyrido[3,4-b]quinoxaline-6-carboxamide obtained in Example 1(2), dry hydrogen chloride gas was passed through the solution for 3 to 5 minutes, and then the crystals which formed were collected by filtration to give the title compound. Pale orange needles, m.p. 262-267°C
Yield; 2.39 g
Elementary Analysis for $C_{20}H_{19}N_5O_5 \cdot 2HCl$
Calcd. (%) C; 55.17, H; 4.86, N; 16.08
Found (%) C; 55.24, H; 5.00, N; 15.97

Example 3

Preparation of N-2-(2-hydroxyethylamino)ethyl 5-hydroxybenzo[f]pyrido[3,4-b]quinoxaline-6-carboxamide:

By the procedure of Example l(2) using 2-(2-aminoethylamino)ethanol in place of N,N-dimethylethylenediamine, the title compound was obtained. Orange needles, m.p. 167-170°C
Yield, 300 mg
Elementary Analysis for $C_{20}H_{19}N_5O_3$
Calcd. (%) C; 63.65, H; 5.07, N; 18.56
Found (%) C; 63.58, H; 5.13, N; 18.54

Example 4

(1) Preparation of ethyl 5-hydroxybenzo[f]pyrido-[4,3-b]quinoxaline-6-carboxylate:

To 20 ml of dimethylformamide was suspended 2.7 g of ethyl 1,4-dihydro-1,4-dioxo-3-hydroxy-2-naphthoate, 3.23 ml of trimethylsilylimidazole was added, and the mixture was stirred at room temperature for 2 hours. To the mixture was added dropwise under ice-cooling a solution of l.0 g of 3,4-diaminopyridine in 20 ml of dimethylformamide. Successively the mixture was stirred under a nitrogen atmosphere for 30 minutes under ice-cooling and then overnight at room temperature. To the reaction mixture was added water, and the mixture was extracted twice with chloroform. The extract was washed, in turn, with dilute hydrochloric acid, a saturated aqueous sodium bicarbonate solution and an aqueous sodium chloride solution, and dried over sodium sulfate. The solvent was evaporated under reduced pressure, and the resulting residue was purified by silica gel column chromatography (eluent; chloroform : ethanol = 10 : 1) and recrystallized from ethanol to give the title compound. Yellow needles, m.p. 185°C
Yield; 1.05 g
Elementary Analysis for $C_{18}H_{13}N_3O_3$
Calcd. (%) C; 67.70, H; 4.11, N; 13.16
Found (%) C; 67.75, H; 4,44, N; 13.28

(2) Preparation of N-β-dimethylaminoethyl 5-hydroxybenzo[f]pyrido[4,3-b]quinoxaline-6-carboxamide:

In 40 ml of benzene was dissolved 700 mg of ethyl 5-hydroxybenzo[f]pyrido[4,3-b]quinoxaline-6-carboxylate obtained by synthesis (1) above, 386 mg of N,N-dimethylethylenediamine was added, and the mixture was refluxed for 30 minutes. After cooling, the crystals which formed were collected by filtration and recrystallized from ethanol to give the title compound. Yellow needles, m.p. 190-191°C
Yield; 561 mg
Elementary Analysis for $C_{20}H_{19}N_5O_2$
Calcd. (%) C; 66.47, H; 5.30, N; 19.38
Found (%) C; 66.62, H; 5.45, N; 19.49

Example 5

(1) Preparation of ethyl 5-hydroxybenzo[f]pyrido[3,2-b]quinoxaline-6-carboxylate:

By the procedure of Example l(l) using 2,3-diaminopyridine in place of 3,4-diaminopyridine, the title compound was obtained. Yellow powder, m.p. 188-190°C
Yield, 4.4 g

(2) Preparation of N-β-dimethylaminoethyl 5-hydroxybenzo[f]pyrido[3,2-b]quinoxaline-6-carboxamide:

To 100 ml of benzene were added consecutively 600 mg of ethyl 5-hydroxybenzo[f]pyrido[3,2-b]quinoxaline-6-carboxylate, 0.2 ml of N,N-dimethylethylenediamine and 10 mg of sodium ethylate, and then the mixture was refluxed for 9 hours. After completion of the reaction, the mixture was evaporated to dryness under reduced pressure, and the residue was recrystallized from ethanol to give the title compound. Orange powder, m.p. 208-211°C (decomposition)
Yield; 650 mg
Elementary Analysis for $C_{20}H_{19}N_5O_2$
Calcd. (%) C; 66.47, H; 5.30, N; 19.38
Found (%) C; 66.21, H; 5.29, N; 19.43

Example 6

Preparation of N-γ-dimethylaminopropyl 5-hydroxybenzo[f]pyrido[3,2-b]quinoxaline-6-carboxamide:

By the procedure of Example 1(2) using ethyl 5-hydroxybenzo[f]pyrido[3,2-b]quinoxaline-6-carboxylate and N,N-dimethyl-1,3-propanediamine, the title compound was obtained. Orange powder, m.p. 175-178°C (decomposition)
Yield, 100 mg
Elementary Analysis for $C_{21}H_{21}N_5O_2$
Calcd. (%) C; 67.18, H; 5.64, N; 18.66
Found (%) C; 67.20, H; 5.76, N; 18.72

Example 7

Preparation of N-2-(2-hydroxyethylamino)ethyl 5-hydroxybenzo[f]pyrido[3,2-b]quinoxaline-6-carboxamide:

By the procedure of Example 1(2) using ethyl 5-hydroxybenzo[f]pyrido[3,2-b]quinoxaline-6-carboxylate and 2-(2-aminoethylamino)ethanol, the title compound was obtained. Orange powder, m.p. 183-190°C
Yield, 700 mg
Elementary Analysis for $C_{20}H_{19}N_5O_3$
Calcd. (%) C; 63.65, H; 5.07, N; 18.56
Found (%) C; 63.62, H; 5.00, N; 18.44

Example 8

(1) Preparation of ethyl 5-hydroxynaphtho[2,1-g]pteridine-6-carboxylate:

By the procedure of Example I(I) using 4,5-diaminopyridine in place of 3,4-diaminopyridine, the title compound was obtained. Orange needles, m.p. 132-133°C
Yield, 1.2 g

(2) Preparation of N-β-dimethylaminoethyl 5-hydroxynaphtho[2,1-g]pteridine-6-carboxamide:

By the procedure of Example I(2) using ethyl 5-hydroxynaphtho[2,1-g]pteridine-6-carboxylate and N,N-dimethylethylenediamine, the title compound was obtained. Orange needles, m.p. 181-182°C
Yield, 550 mg
Elementary Analysis for $C_{19}H_{18}N_6O_2$
Calcd. (%) C; 62.97, H; 5.01, N; 23.19
Found (%) C; 63.01, H; 5.00, N; 23.20

Example 9

Preparation of N-γ-dimethylaminopropyl 5-hydroxynaphtho[2,1-g]pteridine-6-carboxamide:

By the procedure of Example 8(2) using N,N-dimethyl-I,3-propanediamine in place of N,N-dimethylethylenediamine, the title compound was obtained. Orange fine needles, m.p. 105-108°C
Yield, 200 mg
Elementary Analysis for $C_{10}H_{20}N_6O_2$
Calcd. (%) C; 63.82, H; 5.36, N; 22.33
Found (%) C; 63.80, H; 5.34, N; 22.42

Test example

P388 Lymphocytic Leukemia Test

(1) Test animal

Female, $CDF_1$ mice (5-6 weeks old, weighing 17-20 g) were intraperitoneally transplanted with $1 \times 10^6$ cells of p388 lymphocytic leukemia passaged on female, DBA/2 mice on day 0.

(2) Administration route

A suspension of the compound of formula (I) in 0.5% of gum arabic saline solution was administered intraperitoneally once a day, 5 times in all, from day I to day 5, or 2 times in all, day I and day 5. A solution of 5-fluorouracil in saline served as a reference drug, and was administered similarly. Control group were similarly administered with only 0.5% gum arabic saline solution.

Eight animals were used for the treated group with the compound of formula (I) and the reference drug, respectively, and 16 animals for control group.

(3) Evaluation method

The antitumor effect was evaluated according to the criterion of the National Cancer Institute of the United States (NCI). The survivors of each group were recorded for 30 days, and the value of T/C × 100 (%) was calculated from the median survival times for the treated animals (T) and the control animals (C). When the value is 125 or more, the drug is judged to be effective.

(4) Results of test

The results of the test are shown in Table 1.
The compounds of formula (I) of the present invention indicated significant value of T/C × 100.

Table 1

| Compound No. | Dose (mg/kg) | Administration day | T/C × 100 |
|---|---|---|---|
| 1 | 6.3 | 1, 5 | 133 |
| | 12.5 | 1, 5 | 137 |
| | 25.0 | 1, 5 | 275 |
| 5 | 25.0 | 1–5 | 130 |
| | 50.0 | 1, 5 | 135 |
| | 100 | 1, 5 | 195 |
| | 200 | 1, 5 | 200 |
| 7 | 25.0 | 1–5 | 174 |
| | 50.0 | 1–5 | 227 |
| 8 | 25.0 | 1–5 | 133 |
| | 50.0 | 1–5 | 153 |
| | 100 | 1–5 | 148 |
| 5-fluoro-uracil | 3.1 | 1–5 | 136 |
| | 6.3 | 1–5 | 148 |
| | 12.5 | 1–5 | 166 |
| | 25.0 | 1–5 | 176 |

(note)
The compound number shows the number of the Example in which the title compound was prepared.

**Claims for the Contracting States: GB, FR, DE, IT, NL, SE, CH/LI, BE, LU**

1. An azaanthracene compound of the general formula

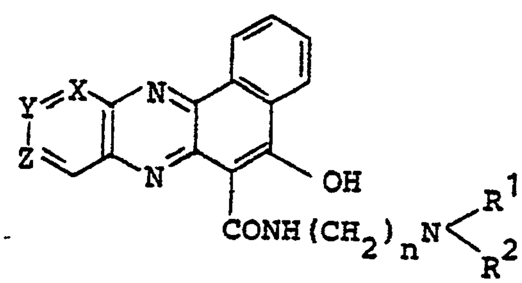

(wherein one or two of X, Y and Z is (are each) a nitrogen atom, and the or each other is CH, $R^1$ and $R^2$ are the same as or different from one another and are each a hydrogen atom, lower alkyl group or β-hydroxyethyl group, and n is 2 or 3) or a salt thereof.

2. An azaanthracene compound according to Claim I, wherein one or two of X, Y and Z is (are each) a nitrogen atom, the or each other is CH, $R^1$ and $R^2$ are both methyl groups, and n is 2.

3. An azaanthracene compound according to Claim I, wherein one or two of X, Y and Z is (are each) a nitrogen atom, the or each other is CH, $R^1$ is a hydrogen atom, $R^2$ is a β-hydroxyethyl group, and n is 2.

4. N-β-dimethylaminoethyl 5-hydroxybenzo[f]pyrido[3,4-b]quinoxaline-6-carboxamide.

5. N-β-dimethylaminoethyl 5-hydroxybenzo[f]pyrido[3,4-b]quinoxaline-6-carboxamide hydrochloride.

6. N-2-(2-hydroxyethylamino)ethyl 5-hydroxybenzo[f]pyrido[3,4-b]quinoxaline-6-carboxamide.

7. N-β-dimethylaminoethyl 5-hydroxybenzo[f]pyrido [4,3-b]quinoxaline-6-carboxamide.

8. N-β-dimethylaminoethyl 5-hydroxybenzo[f]pyrido[3,2-b]quinoxaline-6-carboxamide.

9. N-2-(2-hydroxyethylamino)ethyl 5-hydroxybenzo[f]pyrido[3,2-b]quinoxaline-6-carboxamide.

10. N-β-dimethylaminoethyl 5-hydroxynaphtho[2,1-g]pteridine-6-carboxamide.

11. A method for preparing a compound of the general formula (I), given and defined in claim 1, or salt thereof, which method comprises condensing a compound of the general formula (II)

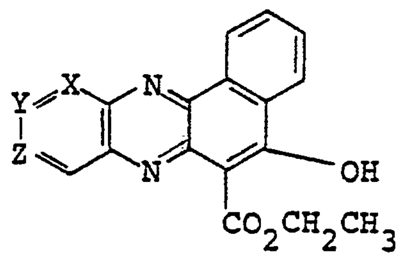

(II)

(wherein X, Y and Z are as defined in claim 1 with an alkylamine of the general formula (IV)

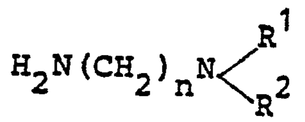

(IV)

(wherein $R^1$, $R^2$ and n are defined in claim 1).

12. A method according to claim 11, wherein the condensation is carried out in an organic solvent at a temperature of from room temperature to 120°C inclusive for a period of from 1 to 24 hours inclusive.

**Claims for the Contracting States: AT, ES, GR**

1. A method for preparing a compound of the general formula (I)

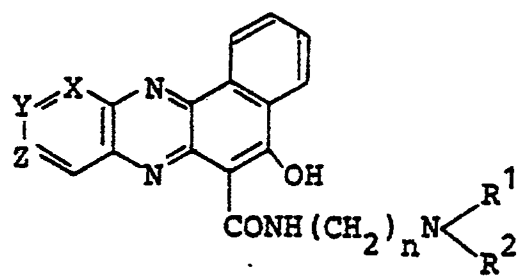

(I)

(wherein one or two of X, Y and Z is (are each) a nitrogen atom, and the or each other is CH, $R^1$ and $R^2$ are the same as or different from one another and are each a hydrogen atom, lower alkyl group or β-hydroxyethyl group, and n is 2 or 3) or a salt thereof, which method comprises condensing a compound of the general formula (II)

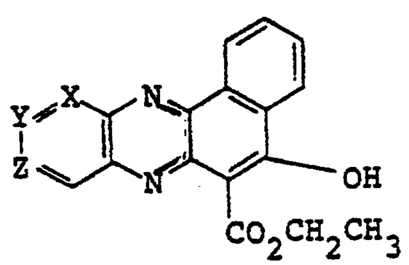

(II)

(wherein X, Y and Z are as defined above with an alkylamine of the general formula (IV)

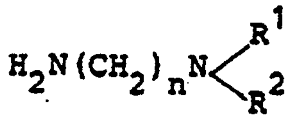

(IV)

(wherein $R^1$, $R^2$ and n are defined above).

2. A method according to Claim I, wherein the condensation is carried out in an organic solvent at a temperature of from room temperature to I20°C inclusive for a period of from I to 24 hours inclusive.

3. A method according to Claim I or Claim 2, wherein, in the compound of the formula (I), one or two of X, Y and Z is (are each) a nitrogen atom, the or each other is CH, $R^1$ and $R^2$ are both methyl groups, and n is 2.

4. A method according to Claim I or Claim 2, wherein, in the compound of the formula (I), one or two of X, Y and Z is (are each) a nitrogen atom, the or each other is CH, $R^1$ is a hydrogen atom, $R^2$ is a β-hydroxyethyl group, and n is 2.

5. A method according to Claim 3, wherein the compound of the formula (I) is N-β- dimethylaminoethyl 5-hydroxybenzo [f]pyrido[3,4-b]quinoxaline-6-carboxamide.

6. A method according to Claim 3, wherein the compound of the formula (I) is N-β-dimethylaminoethyl 5-hydroxybenzo[f]pyrido[3,4-b]quinoxaline-6-carboxamide hydrochloride.

7. A method according to Claim 4, wherein the compound of the formula (I) is N-2- (2-hydroxyethylamino)ethyl 5-hydroxybenzo[f]pyrido[3,4-b]quinoxaline-6-carboxamide.

8. A method according to Claim 3, wherein the compound of the formula (I) is N-β-dimethylaminoethyl 5-hydroxybenzo[f]pyrido[4,3-b]quinoxaline-6-carboxamide.

9. A method according to Claim 3, wherein the compound of the formula (I) is N-β-dimethylaminoethyl 5-hydroxybenzo[f]pyrido[3,2-b]quinoxaline-6-carboxamide.

10. A method according to Claim 4, wherein the compound of the formula (I) is N-2-(2-hydroxyethylamino) ethyl 5-hydroxybenzo[f]pyrido[3,2-b]quinoxaline-6-carboxamide.

11. A method according to Claim 3, wherein the compound of the formula (I) is N-β-dimethylaminoethyl 5-hydroxynaphtho[2,1-g]-pteridine-6-carboxamide.

12. A method for preparing an azaanthracene compound, wherein the said compound has the general formula (I)

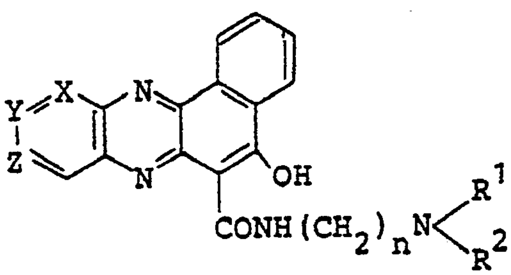

(I)

(wherein one or two of X, Y and Z is (are each) a nitrogen atom, the or each other is CH, $R^1$ and $R^2$ are the same as or different from one another and are each a hydrogen atom, lower alkyl group or β-hydroxyethyl group, and n is 2 or 3) or a salt thereof.

**Revendications pour les Etats contractants: GB, FR, DE, IT, NL, SE, CH, BE, LU**

1. Composé d'azaanthracène répondant à la formule générale suivante:

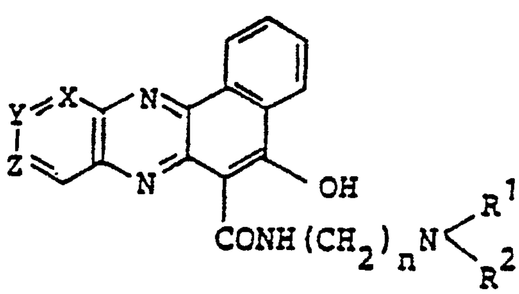

(dans laquelle un ou deux de X, Y et Z est (sont chacun) un atome d'azote, et le ou chaque autre est CH, $R^1$ et $R^2$ sont identiques ou différents l'un de l'autre et sont chacun un atome d'hydrogène, un groupe alkyle inférieur, ou un groupe β-hydroxyéthyle, et n est égal à 2 ou à 3), ou un de leurs sels.

2. Composé d'azaanthracène selon la revendication 1, dans lequel un ou deux de X, Y et Z est (sont chacun) un atome d'azote, le ou chaque autre est CH, $R^1$ et $R^2$ sont tous deux des groupes méthyle, et n est égal à 2.

3. Composé d'azaanthracène selon la revendication 1, dans lequel un ou deux de X, Y et Z est (sont chacun) un atome d'azote, le ou chaque autre est CH, $R^1$ est un atome d'hydrogène, $R^2$ est un groupe β-hydroxyéthyle, et n est égal à 2.

4. N-β-diméthylaminoéthyl 5-hydroxybenzo[f]pyrido-[3,4-b]quinoxaline-6-carboxamide.

5. Chlorhydrate de N-β-diméthylaminoéthyl 5-hydroxybenzo[f]pyrido-[3,4-b]quinoxaline-6-carboxamide.

6. N-2-(2-hydroxyéthylamino)éthyl 5-hydroxybenzo[f]pyrido[3,4-b]quinoxaline-6-carboxamide.

7. N-β-diméthylaminoéthyl 5-hydroxybenzo[f]pyrido-[4,3-b]quinoxaline-6-carboxamide.

8. N-β-diméthylaminoéthyl 5-hydroxybenzo[f]pyrido-[3,2-b]quinoxaline-6-carboxamide.

9. N-2-(2-hydroxyéthylamino)éthyl 5-hydroxybenzo-[f]pyrido[3,2-b]quinoxaline-6-carboxamide.

10. N-β-diméthylaminoéthyl 5-hydroxynaphto[2,1-g]-pétridine-5-carboxamide.

11. Procédé de préparation d'un composé répondant à la formule générale (I), donné et défini en revendication 1, ou un de ses sels, procédé qui comprend l'étape consistant à condenser un composé répondant à la formule générale (II)

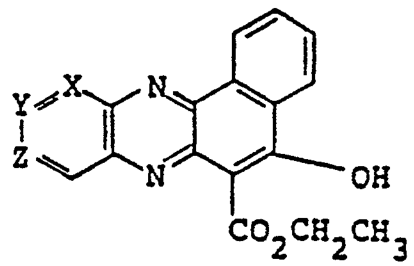

(II)

(dans laquelle X, Y et Z sont tels que définis en revendication 1 avec une alkylamine répondant à la formule générale (IV)

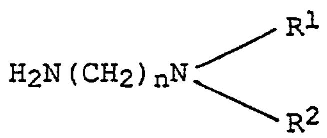

(IV)

(dans laquelle $R^1$, $R^2$ et n sont tels que définis en revendication 1).

12. Procédé selon la revendication 11, dans lequel on effectue la condensation dans un solvant organique à une température comprise entre la température ambiante et 120°C compris pendant une durée allant de 1 à 24 heures comprises.

**Revendications pour les Etats contractants: AT, ES, GR**

1. Procédé pour préparer un composé répondant à la formule générale (I)

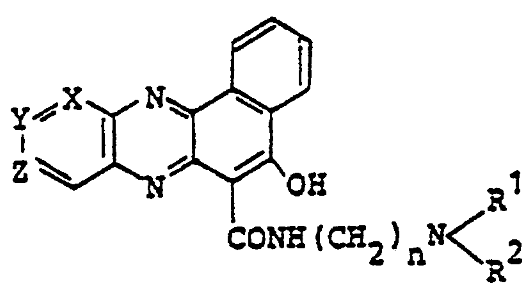

(I)

(dans laquelle un ou deux de X, Y et Z est (sont chacun) un atome d'azote, et le ou chaque autre est CH, $R^1$ et $R^2$ sont identiques ou différents l'un de l'autre et sont chacun un atome d'hydrogène, un groupe alkyle inférieur ou un groupe β-hydroxyéthyle, et n est égal à 2 ou 3) ou un de ses sels, procédé qui comprend l'étape consistant à condenser un composé répondant à la formule générale (II)

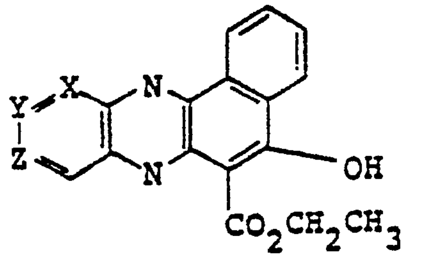

(II)

(dans laquelle X, Y et Z sont tels que définis ci-dessus avec une alkylamine répondant à la formule générale (IV)

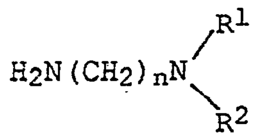

(IV)

(dans laquelle $R^1$, $R^2$ et n sont tels que définis ci-dessus).

2. Procédé selon la revendication 1, dans lequel on exécute la condensation dans un solvant organique à une température comprise entre la température ambiante et 120°C compris pendant une durée allant de 1 à 24 heures comprises.

3. Procédé selon la revendication 1 ou la revendication 2, dans lequel, dans le composé répondant à la formule (I), un ou deux de X, Y et Z est (sont chacun) un atome d'azote, le ou chaque autre est CH, $R^1$ et $R^2$ sont tous deux des groupes méthyle, et n est égal à 2.

4. Procédé selon la revendication 1 ou la revendication 2, dans lequel, dans le composé répondant à la formule (I), un ou deux de X, Y et Z est (sont chacun) un atome d'azote, le ou chaque autre est CH, $R^1$ est un atome d'hydrogène, $R^2$ est un groupe β-hydroxyéthyle, et n est égal à 2.

5. Procédé selon la revendication 3, dans lequel le composé répondant à la formule (I) est le N-β-diméthylaminoéthyl 5-hydroxybenzo[f]pyrido[3,5-b]quinoxaline-6-carboxamide.

6. Procédé selon la revendication 3, dans lequel le composé répondant à la formule (I) est le chlorhydrate de N-β-diméthylaminoéthyl-5-hydroxybenzo[f]pyrido[3,4-b]quinoxaline-6-carboxamide.

7. Procédé selon la revendication 4, dans lequel le composé répondant à la formule (I) est le N-2-(2-hydroxyéthylamino)éthyl 5-hydroxybenzo[f]pyrido[3,4-b]quinoxaline-6-carboxamide.

8. Procédé selon la revendication 3, dans lequel le composé répondant à la formule (I) est le N-β-diméthylaminoéthyl 5-hydroxybenzo[f]pyrido[4,3-b]quinoxaline-6-carboxamide.

9. Procédé selon la revendication 3, dans lequel le composé répondant à la formule (I) est le N-β-diméthylaminoéthyl-5-hydroxybenzo[f]pyrido[3,2-b]quinoxaline-6-carboxamide.

10. Procédé selon la revendication 4, dans lequel le composé répondant à la formule (I) est le N-2-(2-hydroxyéthylamino)éthyl 5-hydroxybenzo[f]pyrido[3,2-b]quinoxaline-6-carboxamide.

11. Procédé selon la revendication 3, dans lequel le composé répondant à la formule (I) est le N–β-diméthylaminoéthyl 5-hydroxynaphto[2,1-g]-pétridine-6-carboxamide.

12. Procédé pour préparer un composé d'azaanthracène, dans lequel le composé répond à la formule générale (I)

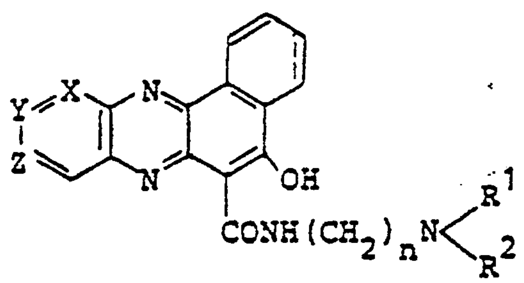

(I)

(dans lequel un ou deux de X, Y et Z est (sont chacun) un atome d'azote, le ou chaque autre est CH, $R^1$ et $R^2$ sont identiques ou différents l'un de l'autre et sont chacun un atome d'hydrogène, un groupe alkyle inférieur ou un groupe β-hydroxyéthyle, et n est égal à 2 ou 3 ou un de ses sels.

**Patentansprüche für die Vertragsstaaten: AT, ES, GR**

1. Verfahren zur Herstellung einer Verbindung der allgemeinen Formel (I),

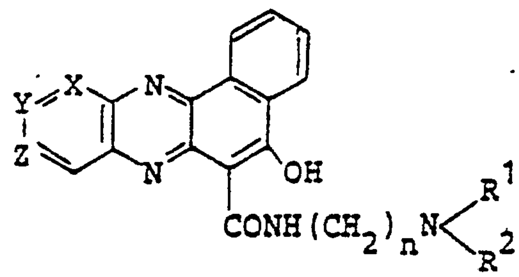

(I)

(worin eine oder zwei der Gruppen X, Y und Z (jeweils) ein Stickstoffatom und die andere oder die anderen CH bedeuten. $R^1$ und $R^2$ gleich oder voneinander verschieden sind und jeweils ein Wasserstoffatom, eine niedrige Alkylgruppe oder eine β-Hydroxyethylgruppe bedeuten und n 2 oder 3 ist) oder ein Salz davon, welches Verfahren das Kondensieren einer Verbindung der allgemeinen Formel (II)

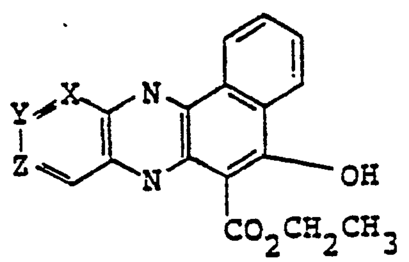

(II)

(worin X, Y und Z wie oben definiert sind) mit einem Alkylamin der allgemeinen Formel (IV)

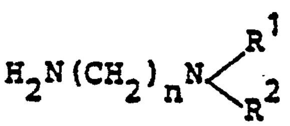

(IV)

(worin $R^1$, $R^2$ und n wie oben definiert sind) umfaßt.

2. Verfahren nach Anspruch 1, worin die Kondensation in einem organischen Lösungsmittel bei einer Temperatur von Raumtemperatur bis inklusive 120°C für einen Zeitraum von 1 bis inklusive 24 h durchgeführt wird.

3. Verfahren nach Anspruch 1 oder 2, worin in der Verbindung der Formel (I) eine oder zwei der Gruppen X, Y und Z (jeweils) ein Stickstoffatom bedeutet und die andere oder die anderen CH bedeuten, $R^1$ und $R^2$ Methylgruppen sind und n 2 ist.

4. Verfahren nach Anspruch 1 oder 2, worin in der Verbindung der Formel (I) eine oder zwei der Gruppen X, Y und Z (jeweils) ein Stickstoffatom bedeutet und die andere oder die anderen CH bedeuten, $R^1$ ein Wasserstoffatom ist, $R^2$ eine β-Hydroxyethylgruppe ist und n 2 ist.

5. Verfahren nach Anspruch 3, worin die Verbindung der Formel (I) N-β-Dimethylaminoethyl-5-hydroxybenzo[f]pyrido-[3,4-b]chinoxalin-6-carboxamid ist.

6. Verfahren nach Anspruch 3, worin die Verbindung der Formel (I) N-β-Dimethylaminoethyl-5-hydroxybenzo[f]pyrido-[3,4-b]chinoxalin-6-carboxamid.Hydrochlorid ist.

7. Verfahren nach Anspruch 3, worin die Verbindung der Formel (I) N-2-(2-Hydroxyethylamino)ethyl-5-hydroxybenzo[f]pyrido[3,4-b]chinoxalin-6-carboxamid ist.

8. Verfahren nach Anspruch 3, worin die Verbindung der Formel (I) N-β-Dimethylaminoethyl-5-hydroxybenzo[f]pyrido-[4,3-b]chinoxalin-6-carboxamid ist.

9. Verfahren nach Anspruch 3, worin die Verbindung der Formel (I) N-β-Dimethylaminoethyl-5-hydroxybenzo[f]pyrido-[3,2-b]chinoxalin-6-carboxamid ist.

10. Verfahren nach Anspruch 3, worin die Verbindung der Formel (I) N-2-(2-Hydroxyethylamino)ethyl-5-hydroxybenzo[f]-pyrido[3,2-b]chinoxalin-6-carboxamid ist.

11. Verfahren nach Anspruch 3, worin die Verbindung der Formel (I) N-β-Dimethylaminoethyl-5-hydroxynaphthol[2,1-g]-pteridin-6-carboxamid ist.

12. Verfahren zur Herstellung einer Azaanthrazenverbindung, worin diese Verbindung die allgemeine Formel (I)

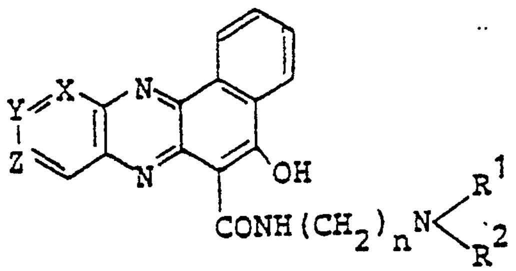

(I)

besitzt (worin eine oder zwei der Gruppen X , Y und Z (jeweils) ein Stickstoffatom und die andere oder die anderen CH bedeuten, $R^1$ und $R^2$ gleich oder voneinander verschieden sind und jeweils ein Wasserstoffatom, eine niedrige Alkylgruppe oder eine β-Hydroxyethylgruppe bedeuten und n 2 oder 3 ist) oder ein Salz davon.

**Patentansprüche für die Vertragsstaaten: GB, FR, DE, IT, NL, SE, CH/LI, BE, LU**

1. Eine Azaanthrazenverbindung der allgemeinen Formel

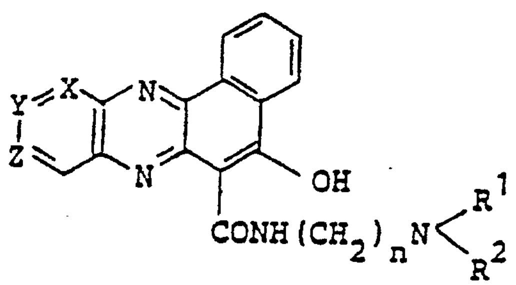

(worin eine oder zwei der Gruppen X, Y und Z (jeweils) ein Stickstoffatom und die andere oder die anderen CH bedeuten, $R^1$ und $R^2$ gleich oder voneinander verschieden sind und jeweils ein Wasserstoffatom, eine niedrige Alkylgruppe oder eine β-Hydroxyethylgruppe bedeuten und n 2 oder 3 ist) oder ein Salz davon.

2. Azaanthrazenverbindung nach Anspruch 1, worin eine oder zwei der Gruppen X, Y und Z (jeweils) ein Stickstoffatom bedeutet und die andere oder die anderen CH bedeuten, $R^1$ und $R^2$ Methylgruppen sind und n 2 ist.

3. Azaanthrazenverbindung nach Anspruch 1, worin eine oder zwei der Gruppen X, Y und Z (jeweils) ein Stickstoffatom bedeutet und die andere oder die anderen CH bedeuten, $R^1$ ein Wasserstoffatom ist, $R^2$ eine β-Hydroxyethylgruppe ist und n 2 ist.

4. N-β-Dimethylaminoethyl-5-hydroxybenzo[f]pyrido[3,4-b]chinoxalin-6-carboxamid.

5. N-β-Dimethylaminoethyl-5-hydroxybenzo[f]pyrido[3,4-b]chinoxalin-6-carboxamid.Hydrochlorid.

6. N-2-(2-Hydroxyethylamino)ethyl-5-hydroxybenzo[f]pyrido[3,4-b]chinoxalin-6-carboxamid.

7. N-β-Dimethylaminoethyl-5-hydroxybenzo[f]pyrido[4,3-b]chinoxalin-6-carboxamid.

8. N-β-Dimethylaminoethyl-5-hydroxybenzo[f]pyrido[3,2-b]chinoxalin-6-carboxamid.

9. N-2-(2-Hydroxyethylamino)ethyl-5-hydroxybenzo[f]pyrido[3,2-b]chinoxalin-6-carboxamid.

10. N-β-Dimethylaminoethyl-5-hydroxynaphtho[2,1-g]pteridin-6-carboxamid.

11. Verfahren zur Herstellung einer Verbindung der allgemeinen Formel (I) nach Anspruch 1 oder eines Salzes davon, welches Verfahren das Kondensieren einer Verbindung der allgemeinen Formel (II)

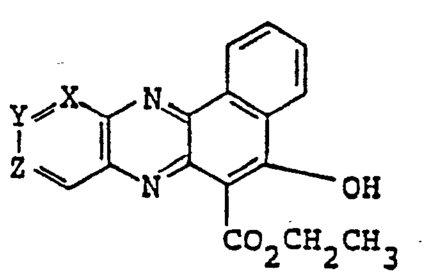

(II)

(worin X, Y und Z wie in Anspruch 1 definiert sind) mit einem Alkylamin der allgemeinen Formel (IV)

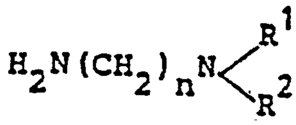 (IV)

(worin $R^1$, $R^2$ und n wie in Anspruch 1 definiert sind) umfaßt.

12. Verfahren nach Anspruch 1, worin die Kondensation in einem organischen Lösungsmittel bei einer Temperatur von Raumtemperatur bis inklusive 120°C für einen Zeitraum von 1 bis inklusive 24 h durchgeführt wird.